(19) Europäisches Patentamt
European Patent Office
Office européen des brevets

(11) Numéro de publication: **0 321 369**
**A1**

# (12) DEMANDE DE BREVET EUROPEEN

(21) Numéro de dépôt: **88420421.5**

(22) Date de dépôt: **15.12.88**

(51) Int. Cl.[4]: **C 07 D 213/75**
C 07 D 239/28,
C 07 D 271/12, A 23 L 1/236

(30) Priorité: **18.12.87 FR 8718114**

(43) Date de publication de la demande:
**21.06.89 Bulletin 89/25**

(84) Etats contractants désignés:
**AT BE CH DE ES FR GB GR IT LI LU NL SE**

(71) Demandeur: **UNIVERSITE CLAUDE BERNARD - LYON 1**
**43, boulevard du 11 Novembre 1918**
**F-69100 Villeurbanne Cédex (FR)**

(72) Inventeur: **Nofre, Claude**
**119 Cours Albert Thomas**
**F-69003 Lyon (FR)**

**Tinti, Jean Marie**
**5 avenue de Grenoble**
**F-69330 Meyzieu (FR)**

**Ouar, Farroudja ép. Chatzopoulos**
**14 rue Raoul Follereau**
**F-42100 Saint Etienne (FR)**

(74) Mandataire: **Laurent, Michel et al**
**Cabinet LAURENT et GUERRE B.P. 32**
**F-69131 Ecully Cedex (FR)**

Revendications pour les Etats contractants suivants: ES + GR.

(54) **Edulcorants derives heterocycliques de N-carbamoyl-, N-thiocarbamoyl- ou N-amidino-glycine ou beta-alanine.**

(57) Agents édulcorants de formule :

$$R\text{-}NH\text{-}\overset{\overset{\displaystyle A}{\|}}{C}\text{-}NH\text{-}(CH_2)_n\text{-}B$$

dans laquelle R est un groupe hétérocyclique qui, dans une forme de réalisation préférée, est un groupe 2-cyanopyrid-5-yle ou 2-cyanopyrimidin-5-yle, A est O, S, NH ou NCN, NH pouvant être salifié sous forme d'hydrochlorure, n est 1 ou 2, B est COOH ou un sel de sodium, potassium, ammonium, calcium ou magnésium.

EP 0 321 369 A1

## Description

### EDULCORANTS DERIVES HETEROCYCLIQUES DE N-CARBAMOYL-, N-THIOCARBAMOYL- OU N-AMIDINO-GLYCINE OU BETA-ALANINE

La présente invention concerne de nouveaux agents édulcorants utiles, notamment, pour édulcorer les aliments, les boissons, les confiseries, les pâtisseries, le chewing gum, les produits d'hygiène, les cosmétiques, les articles de toilette, les produits pharmaceutiques et vétérinaires, et leurs équivalents. Elle concerne aussi des préparations et des compositions contenant de tels agents édulcorants.

Parmi les composés chimiques qui présentent des propriétés édulcorantes, le "suosan" et ses dérivés constituent une série chimique qui a été largement étudiée depuis leur découverte en 1948 par Petersen et Müller (Chem. Ber., 1948, 81, 31-38 ; voir par exemple Beets, Structure-Activity Relationships in Human Chemoreception, Applied Science Publ., London, 1978, pp. 336-337; Crosby et Wingard, in Developments in Sweeteners, Applied Science Publ., London 1979, p. 160 ; Tinti, Nofre et Peytavi, Z. Lebensm. Unters. Forsch., 1982, 175, 266-268). Mais ces composés n'ont jamais été utilisés en pratique car certains d'entre eux libèrent des molécules potentiellement toxiques ; c'est le cas du "suosan" qui conduit à de la 4-nitroaniline. De plus, ces composés sont peu solubles dans les solutions à pH acide (pH 2,5 - 3), dans les conditions donc habituelles d'utilisation des édulcorants de synthèse dans les boissons gazeuses ("soft drinks") qui constituent actuellement le principal débouché des édulcorants.

Dans la demande de brevet EP-A-0195730 on a décrit comme nouveaux agents édulcorants des composés de formule :

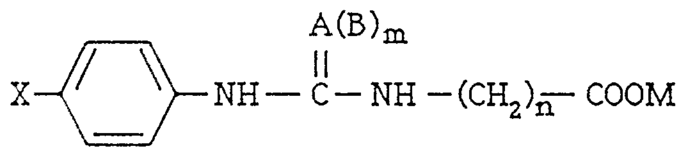

dans laquelle :
- A est un groupe imino, iminium ou méthylène, le groupe iminium pouvant être salifié par un anion organique ou inorganique physiologiquement acceptable ;
- m et n sont des nombres égaux à 1 ou 2 ;
- B :
. quand n = 1, représente H, CN, $OCH_3$, $NO_2$, $SO_2R$, SOR, $SO_2NHR$, $SO_2NR_2$, où R est un groupe alkyle, cycloalkyle ou aryle ayant jusqu'à 10 atomes de carbone, 1 ou 2 atomes de carbone pouvant être substitués par 1 ou 2 atomes de soufre ou d'oxygène ;
. quand n = 2, représente H, CN, $OCH_3$ ;
- X :
. représente CN, $NO_2$, quand B est H, CN, $OCH_3$ ;
. représente CN, $NO_2$, $COCH_3$, CHO, Cl, $CF_3$, F, H, quand B est $NO_2$, $SO_2R$, SOR, $SO_2NHR$, $SO_2NR_2$ ;
- M est un atome d'hydrogène ou un cation organique ou inorganique physiologiquement acceptable.

Comme c'était déjà le cas pour le suosan et ses dérivés, les composés décrits dans cette demande de brevet sont trop peu solubles dans l'eau à pH acide (pH 2,5 - 3) pour que l'on puisse envisager leur utilisation dans les boissons gazeuses ("soft drinks"), principal débouché des édulcorants de synthèse.

La présente invention concerne des agents édulcorants de formule :

A
||
R-NH- C-NH-$(CH_2)_n$-B

dans laquelle :
- A est O, S, NH ou N-CN, NH pouvant être salifié par un acide organique ou inorganique physiologiquement acceptable ;
- n est 1 ou 2 ;
- B est un groupe COOH ou un sel de sodium, potassium, ammonium, calcium ou magnésium ;

et caractérisés en ce que R est un groupe hétérocyclique, monocyclique ou bicyclique,
. le groupe monocyclique étant choisi dans le groupe constitué par les radicaux :

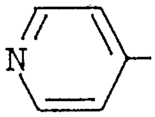

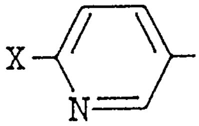

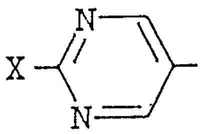

dans lesquels X est :
Cl,
CN et
$NO_2$,
. le groupe bicyclique étant choisi dans le groupe constitué par les radicaux :

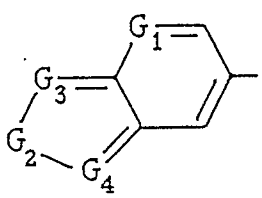

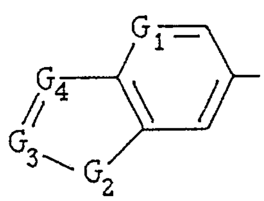

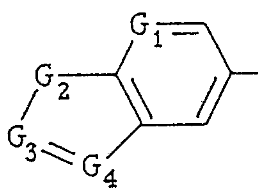

dans lesquels :
$G_1$ est CH ou N,
$G_2$ est $CH_2$, CO, NH, $NCH_3$, O ou S,
$G_3$ et $G_4$ sont CH, $CCH_3$, N ou NO.

En d'autres termes, les composés de la présente invention se distinguent du suosan ou de ses dérivés et des composés décrits dans la demande de brevet EP-A-0195730 par le remplacement du groupe carbocyclique X-$C_6H_4$ par un groupe hétérocyclique R, ce qui a pour effet d'accroître leur solubilité dans l'eau en milieu acide, aux pH donc d'utilisation des boissons gazeuses (pH 2,5 -3). De plus, le remplacement d'un groupe carbocyclique X-$C_6H_4$ par un groupe hétérocyclique R ne modifie que peu le pouvoir édulcorant des composés, ce qui est totalement inattendu quand on sait que toute modification, même légère, de la structure

moléculaire d'un agent édulcorant entraîne souvent une suppression du caractère édulcorant, les relations entre la structure et l'activité édulcorante étant en effet imprévisibles (M.G.J. BEETS, Structure-Activity Relationships in Human Chemoreception, Applied Science Publ., London, 1978, p. 259-362 ; H. VAN DER WEL, A. VAN DER HEIJDEN, H. G. PEER, Food Reviews International, 1987, 3, 193-268).

Avantageusement, dans les agents édulcorants selon l'invention :

- R est un groupe 2-cyanopyrid-5-yle ou 2-cyanopyrimidin-5-yle ;
- A est O ou NH, NH pouvant être salifié par HCl ;
- n est 1 quand A est NH et n est 2 quand A est 0.

L'invention concerne également le procédé qui consiste à édulcorer les aliments, boissons, confiseries, pâtisseries, chewing gum, produits d'hygiène, cosmétiques, articles de toilette, produits pharmaceutiques et vétérinaires, et leurs équivalents, en leur ajoutant une quantité adéquate d'un ou plusieurs agents édulcorants selon la présente invention. Par "quantité adéquate", on désigne une quantité d'agent édulcorant suffisante pour produire la perception d'une saveur sucrée.

L' invention concerne aussi les préparations édulcorées suivant le procédé de la présente invention.

L'invention concerne également les compositions édulcorantes caractérisées en ce qu'elles comprennent une quantité adéquate d'au moins un agent édulcorant selon la présente invention, et un support ou agent de charge approprié.

L'invention concerne aussi les compositions édulcorantes caractérisées en ce qu'elles comprennent une quantité adéquate d'un ou plusieurs agents édulcorants selon la présente invention, et un ou plusieurs autres agents édulcorants.

Les produits susceptibles d'être édulcorés par les agents édulcorants de la présente invention comprennent tous les produits pour lesquels on désire un composant de goût sucré, notamment, et ce de manière non limitative, les produits alimentaires (pour la consommation humaine ou animale), les boissons (boissons alcooliques, boissons non alcooliques, jus, boissons gazeuses), les confiseries, les pâtisseries, le chewing gum, les produits d'hygiène, les cosmétiques, les produits pharmaceutiques et vétérinaires, et leurs équivalents.

Les agents édulcorants de la présente invention peuvent être ajoutés sous forme pure aux produits comestibles pour leur communiquer un goût sucré. Toutefois, par suite du pouvoir sucrant élevé des présents agents édulcorants, ils sont généralement mélangés à un support ("carrier") ou à un agent de charge approprié ("bulking agent"). Avantageusement, les supports ou agents de charge appropriés sont choisis dans le groupe constitué par le polydextrose, l'amidon, les maltodextrines, la cellulose, la méthylcellulose, la carboxyméthylcellulose, l'hydroxyméthylcellulose, la cellulose microcristalline, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents.

Les présents agents édulcorants peuvent, dans un produit comestible, être employés seuls, comme unique agent édulcorant, ou sous forme de mélanges de deux ou plusieurs agents édulcorants de la présente invention. Les présents agents édulcorants peuvent, en outre, être utilisés en combinaison avec d'autres agents édulcorants tels que les sucres (saccharose), le sirop de maïs, le fructose, les dérivés dipeptidiques sucrés (aspartame, alitame), la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium ou calcium, l'acide cyclamique et ses sels de sodium, potassium, ammonium ou calcium, le trichlorogalactosucrose, la monelline, la thaumatine, et leurs équivalents.

Le procédé de préparation des agents édulcorants de la présente invention varie suivant que A, dans la formule de l'agent édulcorant que l'on veut obtenir, est O, S, NH ou NCN.

Le procédé consiste à faire réagir :

(1) $RNH_2$ et $A=C=N\text{-}(CH_2)_n\text{-}B$

quand A est O, la réaction étant effectuée dans un solvant organique comme l'acétonitrile ou le chloroforme à température ambiante ou à ébullition, ou

(2) $R\text{-}N=C=A$ et $H_2N\text{-}(CH_2)_n\text{-}COOH$

quand A est O ou S, la réaction étant effectuée dans l'eau à température ambiante, $R\text{-}N=C=A$ étant préalablement dissous dans un solvant organique comme le benzène ou le chlorobenzène, ou

(3) $R\text{-}NH\text{-}CS\text{-}NH_2$ et $H_2N\text{-}(CH_2)_n\text{-}B$ ou

$R\text{-}NH\text{-}CS\text{-}NH\text{-}(CH_2)_n\text{-}B$ et $NH_3$

quand A est NH, ou

$R\text{-}NH\text{-}CS\text{-}NH\text{-}(CH_2)_n\text{-}B$ et $H_2NCN$

quand A est NCN, les réactions étant effectuées à température ambiante ou à l'ébullition en présence d'un agent de condensation comme la dicyclohexylcarbodiimide, ou

(4) $R\text{-}NH=CCl\text{-}NH\text{-}(CH_2)_n\text{-}B$ et $NH_3$

quand A est NH, $R\text{-}NH=CCl\text{-}NH\text{-}(CH_2)_n\text{-}B$ étant obtenu par condensation entre $R\text{-}N=CCl_2$ et $H_2N\text{-}(CH_2)_n\text{-}B$ dans le chloroforme ou l'acétate d'éthyle à température ambiante, le milieu réactionnel étant porté à 70° C pour la condensation avec $NH_3$, ou

(5) $R\text{-}NH\text{-}C(=A)\text{-}SCH_3$ et $H_2N\text{-}(CH_2)_n\text{-}COOH$

quand A est NH ou NCN, la réaction étant effectuée dans un mélange éthanol-eau à l'ébullition en présence d'une base comme NaOH ou $N(C_2H_5)_3$, ou

(6) R-NH-C(=NCN)-NH-$(CH_2)_n$-B et HCl dans l'eau

quand A est NH, l'hydrolyse étant effectuée à 70.C.

Dans les formules des réactifs utilisés, R et n correspondent aux définitions données précédemment, B correspond à un groupe carboxyle protégé, sous forme par exemple d'un ester (ester méthylique, éthylique, tert-butylique, benzylique), le groupe protecteur étant par la suite éliminé par le moyen le plus approprié qui peut être, par exemple, une saponification par NaOH ou une hydrolyse par HCl.

Pour obtenir les composés de l'invention, le choix entre les procédés (1) à (6) sera fait par l'homme de l'art en fonction de la nature de A, R et B, de la valeur de n et des conditions expérimentales propres à chacun des procédés.

Les composés de l'invention peuvent exister sous forme acide ou sous forme de sel. Ils peuvent donc être salifiés par des bases inorganiques ou organiques physiologiquement acceptables, ou, dans les composés où A est NH, par des acides inorganiques ou organiques physiologiquement acceptables. L'une des méthodes de choix pour préparer ces sels consiste à concentrer à sec sous vide un mélange, en solution aqueuse, d'un composé de l'invention et d'un équivalent d'une base ou d'un acide inorganique ou organique. Les sels préférés de l'invention sont les sels de sodium, potassium, ammonium, calcium et magnésium ou, quand A est NH, les hydrochlorures.

La purification des composés de l'invention a été réalisée selon les techniques standards (recristallisation, chromatographie). Leur pureté et leur structure ont été vérifiées par les techniques classiques (chromatographie sur couche mince, chromatographie liquide haute performance, spectrométrie infra-rouge, résonance magnétique nucléaire, analyse élémentaire).

Le pouvoir édulcorant des composés ainsi préparés a été évalué par un groupe de huit goûteurs expérimentés. Pour cela, les composés, en solution aqueuse à des concentrations variables, sont comparés, sur le plan gustatif, à une solution témoin de saccharose à 2 % et dans certains cas à 5 % et 10 %, c'est-à-dire à des concentrations correspondant à celles utilisées lors d'un usage courant. Le pouvoir sucrant des édulcorants de synthèse varie en effet suivant la concentration de la solution de saccharose utilisée comme référence. Le pouvoir édulcorant du composé testé par rapport au saccharose correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de goûteurs, avoir la même intensité édulcorante.

Les agents édulcorants de la présente invention ont pour utilité de pouvoir être ajoutés à tout produit comestible dans lequel on désire apporter un goût sucré, à condition qu'on les ajoute en proportions suffisantes pour atteindre le niveau d'édulcoration désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits, le profil gustatif des produits comestibles et le niveau d'édulcoration désiré. Toute personne du métier peut facilement déterminer la proportion optimale d'agent édulcorant qui doit être employée pour l'obtention d'un produit comestible en réalisant des analyses sensorielles de routine. Les agents édulcorants préférés de la présente invention sont, en général, ajoutés aux produits comestibles dans des proportions d'environ 0,001 à environ 0,02 pour cent en poids du produit comestible. Les produits concentrés contiendront évidemment des pourcentages plus élevés d'agent(s) édulcorant(s), et seront ensuite dilués suivant les intentions finales d'utilisation.

Un avantage très important des agents édulcorants de la présente invention est de donner souvent une saveur sucrée très proche de celle du saccharose, notamment sans arrière-goût de réglisse (comme c'est le cas par exemple pour la glycyrrhizine ou la thaumatine) et sans arrière-goût métallique ou amer (comme c'est le cas par exemple pour la saccharine ou l'acésulfame-K).

Les dérivés hétérocycliques décrits dans la présente invention, tout en ayant une stabilité en milieu acide comparable à celle de leurs analogues carbocycliques décrits dans l'art antérieur (Z. Lebensm. Unters. Forsch., 1982, 175, 266-268 et brevet EP-A-0195730) ont l'avantage, comme déjà dit, d'être beaucoup plus solubles dans l'eau, comme l'étude comparée de leur solubilité a pu le montrer.

La solubilité a été mesurée de manière suivante. On met en suspension dans l'eau à pH 3 (tampon phosphate) et à température voisine de 0° C (ce qui correspond aux conditions de préparation et de stockage des boissons gazeuses) un excès du composé dont veut déterminer la solubilité. On soumet alors la suspension, toujours dans un bain de glace, à l'action des ultrasons pendant 60 min, puis on filtre (sur filtre Millipore Millex-HV 0,45 micromètre). Le composé, qui se trouve ainsi dissous à saturation dans le filtrat, est dosé par chromatographie liquide haute performance (HPLC) par rapport à une solution de référence de concentration connue.

C'est ainsi qu'on a, par exemple, observé que le composé décrit dans l'exemple 4 de la présente invention (composé dans lequel R est un groupe 2-cyanopyrid-5-yle) a une solubilité à 0° C, d'environ 400 mg/l, pendant que, dans les mêmes conditions, son analogue carbocyclique 4-cyanophényle (exemple 16 décrit dans Z. Lebensm. Unters. Forsch., 1982, 175, 266-268) n'a qu'une solubilité d'environ 100 mg/l, la solubilité du dérivé hétérocyclique étant donc environ 4 fois plus élevée que celle de son analogue carbocylique. De même, on a observé par exemple que le composé décrit dans l'exemple 10 de la présente invention (composé dans lequel R est un groupe 5-benzofurazanyle) a une solubilité, à 0° C, d'environ 1150 mg/l, soit environ 2,8 fois plus que celle du composé carbocyclique 4-nitrophényle (exemple 3 du brevet EP-A-0195730) qui est d'environ 410 mg/l.

La manière dont l'invention peut être réalisée et les avantages qui en découlent ressortiront mieux des exemples de réalisation qui suivent.

EXEMPLE 1 :

Synthèse de la 4-pyridylcarbamoyl-bêta-alanine :

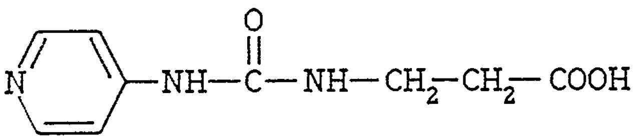

Une solution de 1 g (10,64 mmol) de 4-aminopyridine et de 2,3 g (15,9 mmol) de bêta-alanine éthyl ester isocyanate dans 30 ml d'acétonitrile sec est agitée durant 20 h à température ambiante. Après concentration à sec et trituration dans l'éther (4 x 20 ml) du résidu huileux résultant, 2,5 g (rendement 88 %) de 4-pyridylcarbamoyl-bêta-alanine éthyl ester sont obtenus sous forme d'un solide dont le point de fusion est de 95°C.

Une solution de 2 g (8,4 mmol) de 4-pyridyl carbamoyl-bêta-alanine éthyl ester et de 0,37 g (9,2 mmol) d'hydroxyde de sodium dans 50 ml de méthanol et 0,1 ml d'eau est maintenue sous agitation 24 h à température ambiante. Après concentration à sec, le résidu huileux résultant est repris dans 15 ml d'eau. Après lavage par 3 x 10 ml de dichlorométhane, la phase aqueuse est acidifiée par une solution d'acide chlorhydrique 3N jusqu'à l'obtention d'un pH voisin de 3. Le précipité formé est filtré, lavé par 10 ml d'eau froide puis séché. 1,36 g (rendement 95 %) de 4-pyridylcarbamoyl-bêta-alanine sont obtenus sous forme d'un solide dont le point de fusion est de 222°C.

Le pouvoir édulcorant de ce composé correspond approximativement, sur une base pondérale, à 50 (cinquante) fois celui du saccharose (par comparaison avec une solution de saccharose à 2%).

EXEMPLE 2 :

Synthèse de la 2-chloro-5-pyridylcarbamoyl-bêta-alanine :

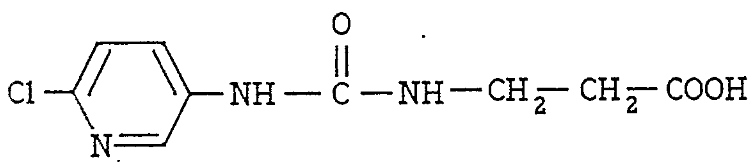

A une solution de 25 ml d'eau contenant 1,26 g (14,2 mmol) de bêta-alanine et 0,75 g (7 mmol) de carbonate de sodium est ajoutée une solution de 2 g (12,9 mmol) de 2-chloro-5-pyridyl isocyanate dissous dans 25 ml de benzène. Une agitation vigoureuse est maintenue durant 1 heure à température ambiante avant de procéder à l'extraction du mélange par 3 x 50 ml d'éther éthylique. La phase aqueuse est refroidie puis acidifiée par une solution d'acide chlorhydrique 3 N jusqu'à l'obtention d'un pH d'environ 3. Le précipité formé est filtré, lavé par 10 ml d'eau froide puis séché. 1,5 g (rendement 45 %) de 2-chloro-5-pyridylcarbamoyl-bêta-alanine sont obtenus sous forme d'un solide dont le point de fusion est de 200°C.

Le pouvoir édulcorant de ce composé correspond approximativement, sur une base pondérale, à 50 (cinquante) fois celui du saccharose (par comparaison avec une solution de saccharose à 2 %).

EXEMPLE 3 :
Synthèse de l'acide N-[2-chloro-5-aminopyridyl(imino)méthyl]-2-aminoéthanoïque :

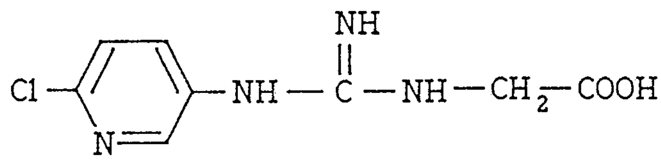

Une solution de 1,2 g (6,4 mmol) de 2-chloro-5-pyridyl thiourée, de 1 g (7,68 mmol) de glycine tert-butyl ester et de 1,45 g (7,04 mmol) de dicyclohexylcarbodiimide dans 30 ml d'acétate d'éthyle est chauffée 2 h 30 à 60° C. Le précipité formé de dicyclohexylthiourée est éliminé par filtration. Le filtrat est concentré à sec. Le produit obtenu est repris dans 20 ml de dichlorométhane, puis extrait par une solution aqueuse d'acide chlorhydrique 0,25N (4 x 30 ml). La solution acide est ensuite neutralisée avec une solution 1N d'hydroxyde de sodium, puis extraite par le dichlorométhane (3 x 20 ml), ce qui permet d'obtenir, sous forme d'une huile, 0,65 g (rendement 36 %) de N-[2-chloro-5-aminopyridyl(imino)méthyl]-2-aminoéthanoate de tert-butyle.

0,65 g (2,3 mmol) de l'ester ainsi obtenu sont dissous dans 1,5 ml d'acide acétique glacial et 3,2 ml (2,3 mmol) d'une solution 7N d'acide chlorhydrique dans le dioxane. Après 1 h 30 à température ambiante, les solvants sont éliminés sous pression réduite. Le résidu est repris dans l'éther éthylique (4 x 20 ml) puis dissous dans 15 ml d'une solution aqueuse saturée de carbonate de sodium. Après lavage par le dichlorométhane (3 x 10 ml), la phase aqueuse est acidifiée avec l'acide chlorhydrique 3N jusqu'à l'obtention d'un pH 4. Le précipité formé est filtré puis est lavé par 5 ml d'eau et séché. 0,11 g (rendement 21 %) d'acide N-[2-chloro-5-aminopyridyl(imino)méthyl]-2-aminoéthanoïque sont obtenus sous forme d'un solide dont le point de fusion est de 240° C.

Le pouvoir édulcorant de ce composé correspond approximativement, sur une base pondérale, à 50 (cinquante) fois celui du saccharose (par comparaison avec une solution de saccharose à 2 %).

EXEMPLE 4 :

Synthèse de la 2-cyano-5-pyridylcarbamoyl-bêta-alanine :

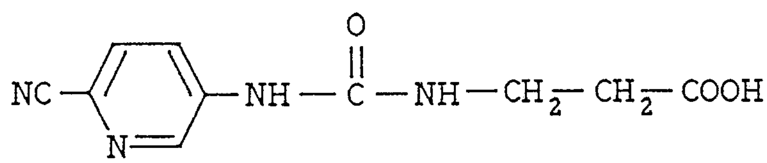

Ce composé est obtenu, sous forme monohydratée, à partir de la bêta-alanine et du 2-cyano-5-pyridyl isocyanate, en suivant le protocole expérimental décrit dans l'exemple 2 (rendement 24 %, point de fusion 173° C).

Le pouvoir édulcorant de ce composé correspond approximativement, sur une base pondérale, à 700 (sept cents) fois celui du saccharose (par comparaison avec une solution de saccharose à 2 %).

EXEMPLE 5 :

Synthèse de la 2-cyano-5-pyrimidinylcarbamoyl-bêta-alanine :

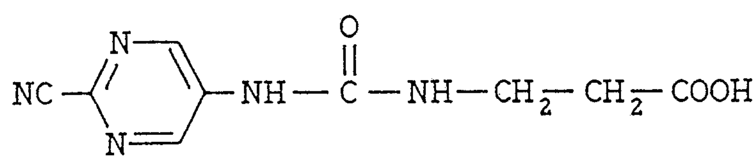

Une solution de 0,83 g (7 mmol) de 2-cyano-5-aminopyrimidine et de 1 g (7 mmol) de bêta-alanine éthyl ester

isocyanate dans 30 ml d'acétonitrile sec est chauffée durant 4 h à 70° C. Le mélange réactionnel est concentré à sec et le résidu est directement soumis à une hydrolyse par HCl 0,1N (50 ml) durant 3 h à 70°C. Après refroidissement, la solution est traitée par NaOH jusqu'à obtention d'un pH 10, est lavée par 3 x 40 ml de dichlorométhane, est acidifiée par HCl jusqu'à obtention d'un pH 3, puis est concentrée à sec sous vide. Le résidu est traité par l'éthanol absolu, ce qui permet d'obtenir, par concentration à sec de l'extrait éthanolique, 0,74g (rendement 45 %) de 2-cyano-5-pyrimidinylcarbamoyl-bêta-alanine sous forme d'un solide dont le point de fusion est de 183° C.

Le pouvoir édulcorant de ce composé correspond approximativement, sur une base pondérale, à 400 (quatre cents) fois celui du saccharose (par comparaison avec une solution de saccharose à 2 %).

EXEMPLE 6 :

Synthèse de la 2-cyano-5-pyrimidinylthiocarbamoyl-bêta-alanine :

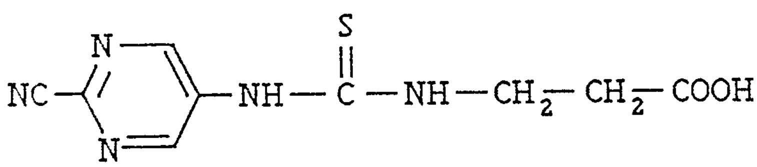

Ce composé est obtenu à partir de la bêta-alanine et du 2-cyano-5-pyrimidinyl isothiocyanate, en suivant le protocole expérimental décrit dans l'exemple 2 (rendement 13 %, point de fusion 135 °C).

Le pouvoir édulcorant de ce composé correspond approximativement, sur une base pondérale, à 1 000 (mille) fois celui du saccharose (par comparaison avec une solution de saccharose à 2 %).

EXEMPLE 7 :

Synthèse de la 5-benzofurazanylcarbamoyl glycine :

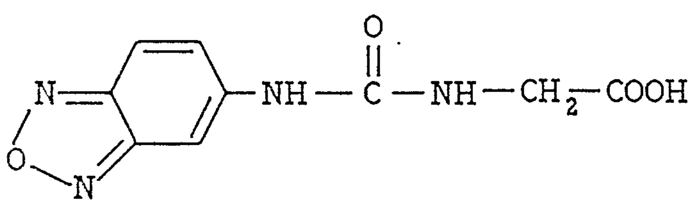

Ce composé est obtenu à partir de la glycine et du 5-benzofurazanyl isocyanate en suivant le protocole expérimental décrit dans l'exemple 2 (rendement 68 %, point de fusion 212°C).

Le pouvoir édulcorant de ce composé correspond approximativement, sur une base pondérale, à 20 (vingt) fois celui du saccharose (par comparaison avec une solution de saccharose à 2 %).

EXEMPLE 8 :

Synthèse de la 5-benzofurazanylcarbamoyl-bêta-alanine :

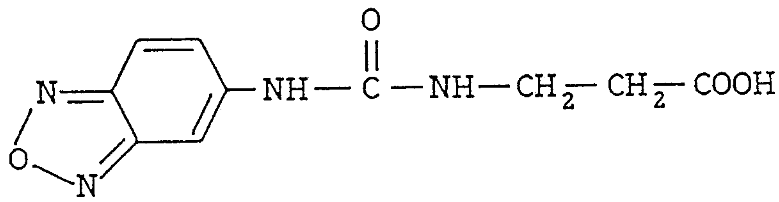

Ce composé est obtenu à partir de la bêta-alanine et du 5-benzofurazanyl isocyanate, en suivant le protocole expérimental décrit dans l'exemple 2 (rendement 47 %, point de fusion 208 °C).

Le pouvoir édulcorant de ce composé correspond approximativement, sur une base pondérale, à 800 (huit cents) fois celui du saccharose (par comparaison avec une solution de saccharose à 2 %).

EXEMPLE 9 :

Synthèse de l'acide N-[5-benzofurazanylamino(imino)méthyl]-2-aminoéthanoïque :

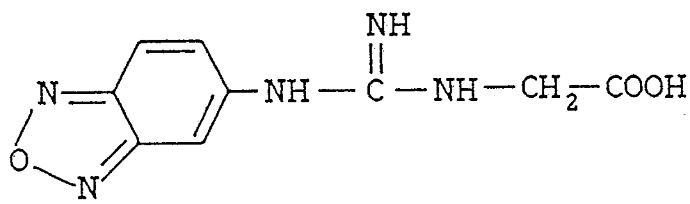

Ce composé est obtenu à partir de la 5-benzofurazanyl thiourée et de la glycine tert-butyl ester, en suivant le protocole expérimental décrit dans l'exemple 3 (rendement 25 %, point de fusion 181°C).

Le pouvoir édulcorant de ce composé correspond approximativement, sur une base pondérale, à 200 (deux cents) fois celui du saccharose (par comparaison avec une solution de saccharose à 2 %).

EXEMPLE 10 :

Synthèse de l'acide N-[cyanoimino(5-benzofurazanylamino)méthyl]-3-aminopropanoïque :

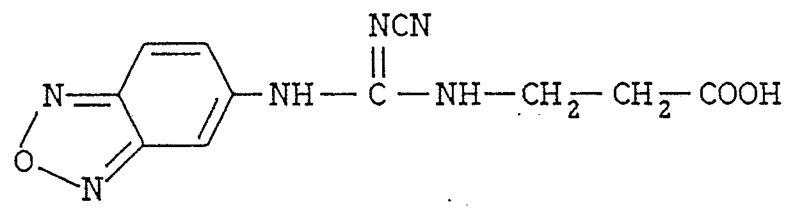

Une solution de 1,3 g (4,4 mmol) de 5-benzofurazanyl thiocarbamoyl-bêta-alanine éthyl ester, de 0,37 g (8,8 mmol) de cyanamide, de 1,36 g (6,6 mmol) de dicyclohexylcarbodiimide et de 0,1 ml de triéthylamine dans 30 ml d'acétonitrile sec est laissée 40 heures à température ambiante. Le mélange est concentré à sec. L'huile résultante est triturée dans l'éther éthylique (3 x 30 ml). On obtient 1 g (rendement 76 %) d'un solide qui, purifié sur colonne de silice (éluant $CHCl_3$ 90/acétone 10) conduit à 0,5 g de N-[cyanoimino(5-benzofurazanylamino)méthyl]-3-aminopropanoate d'éthyle (point de fusion 155°C).

Une solution de 0,5 g (1,65 mmol) de l'ester ainsi obtenu et de 0,066 g (1,65 mmol) d'hydroxyde de sodium dans 15 ml de méthanol et 0,1 ml d'eau est laissée à température ambiante pendant 30 h. Le mélange est concentré à sec. Le résidu obtenu est additionné de 10 ml d'eau et purifié par lavage par le dichlorométhane (3 x 20 ml). La phase aqueuse refroidie est acidifiée avec une solution d'acide chlorhydrique 3N jusqu'à l'obtention d'un pH voisin de 3. Le solide obtenu est récupéré par filtration puis lavé par 2 x 3 ml d'eau. Après séchage, 0,2 g (rendement 44 %) d'acide N-[cyanoimino-(5-benzofurazanylamino)méthyl]-3-aminopropanoï-

que sont obtenus (point de fusion 92°C).

Le pouvoir édulcorant de ce composé correspond approximativement, sur une base pondérale, à 200 (deux cents) fois celui du saccharose (par comparaison avec une solution de saccharose à 2 %).

EXEMPLE 11 :

Synthèse de la 1-oxyde-5-benzofurazanylcarbamoyl-bêta-alanine :

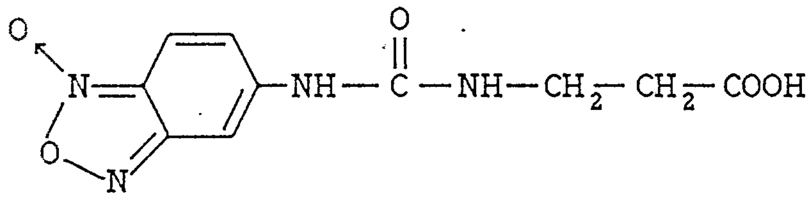

Ce composé est obtenu à partir de la bêta-alanine et du 1-oxyde-5-benzofurazanyl isocyanate, en suivant le protocole expérimental décrit dans l'exemple 2 (rendement 76 %, point de fusion 196°C).

Le pouvoir édulcorant de ce composé correspond approximativement, sur une base pondérale, à 500 (cinq cents) fois celui du saccharose (par comparaison avec une solution de saccharose à 2 %).

EXEMPLE 12 :

Synthèse de la 5-benzothiofurazanylcarbamoyl-bêta-alanine :

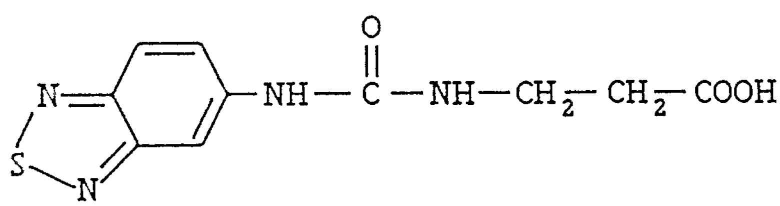

Ce composé est obtenu à partir de la bêta-alanine et du 5-benzothiofurazanyl isocyanate, en suivant le protocole expérimental décrit dans l'exemple 2 (rendement 47 %, point de fusion 189°C).

Le pouvoir édulcorant de ce composé correspond approximativement, sur une base pondérale, à 50 (cinquante) fois celui du saccharose (par comparaison avec une solution de saccharose à 2 %).

EXEMPLE 13 :

Synthèse de la (1H-indazol-6-yl)thiocarbamoyl-bêta-alanine :

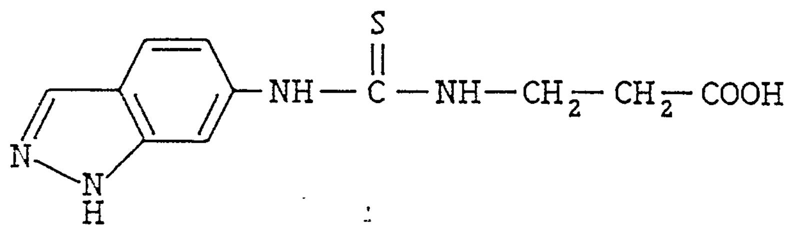

Une solution de 5 g (28,6 mmol) de 1H-indazol-6-yl isothiocyanate, de 5,3 g (34,3 mmol) de bêta-alanine

éthyl ester hydrochlorure, et de 4,8 ml (34,3 mmol) de triéthylamine dans 50 ml de chloroforme est laissée à température ambiante sous agitation durant une heure. La phase chloroformique est lavée par l'eau (2 x 10 ml) et par une solution d'acide chlorhydrique 1N (2 x 10 ml). Elle est séchée et concentrée à sec. Le résidu (8 g) est trituré dans l'éther éthylique (3 x 20 ml). On obtient 3,1 g de (1H-indazol-6-yl)thiocarbamoyl-bêta-alanine éthyl ester (rendement 28 %, point de fusion 145° C).

Une solution de 0,6 g (2,05 mmol) de l'ester ainsi obtenu et de 0,09 g (2,36 mmol) d'hydroxyde de sodium dans 15 ml de méthanol et 0,1 ml d'eau est laissée à température ambiante et sous agitation pendant 20 h. Après concentration à sec, le résidu obtenu est additionné de 10 ml d'eau. Après purification par extraction par l'acétate d'éthyle (3 x 20 ml), la phase aqueuse est refroidie, puis acidifiée par une solution d'acide chlorhydrique 3N jusqu'à l'obtention d'un pH voisin de 3. 0,293 g (rendement 56 %) de (1-H-indazol-6-yl)-thiocarbamoyl-bêta-alanine sont obtenus sous forme d'un solide dont le point de fusion est de 175° C.

Le pouvoir édulcorant de ce composé correspond approximativement, sur une base pondérale, à 150 (cent cinquante) fois celui du saccharose (par comparaison avec une solution de saccharose à 2 %).

Les pouvoirs édulcorants obtenus avec les différents composés cités dans les Exemples 1 à 13 sont récapitulés dans le Tableau I ci-après ; les pouvoirs édulcorants ainsi donnés ont été évalués, sur une base pondérale, par rapport à une solution de saccharose à 2 %.

TABLEAU I

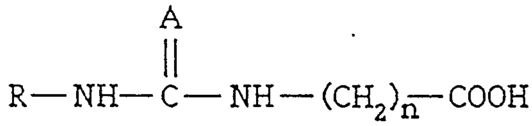

| Composé | R | A | n | Pouvoir édulcorant |
|---|---|---|---|---|
| 1 | 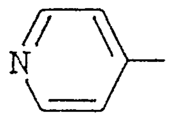 | O | 2 | 50 |
| 2 | 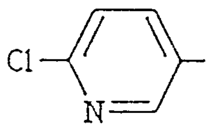 | O | 2 | 50 |
| 3 | 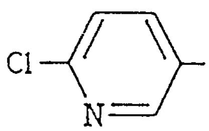 | NH | 1 | 50 |
| 4 | 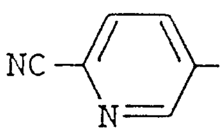 | O | 2 | 700 |
| 5 | 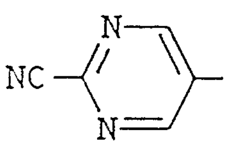 | O | 2 | 400 |
| 6 | 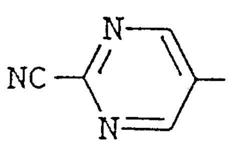 | S | 2 | 1 000 |

| Composé | R | A | n | Pouvoir édulcorant |
|---|---|---|---|---|
| 7 | 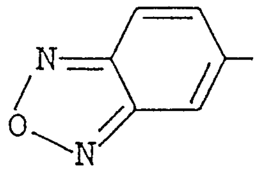 | O | 1 | 20 |
| 8 | 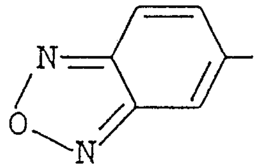 | O | 2 | 800 |
| 9 | 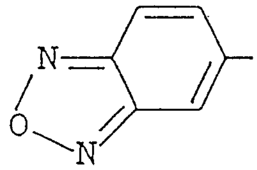 | NH | 1 | 200 |
| 10 | 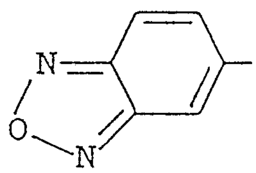 | NCN | 2 | 200 |
| 11 | 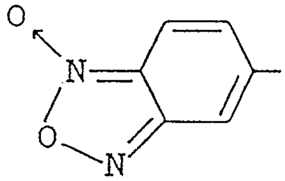 | O | 2 | 500 |
| 12 | 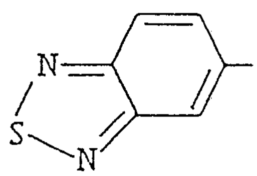 | O | 2 | 50 |
| 13 | 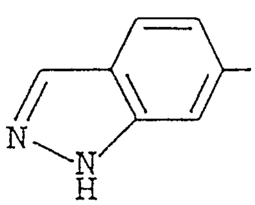 | S | 2 | 150 |

**Revendications**

1. Agents édulcorants de formule :

A
||
R-NH- C -NH-$(CH_2)_n$-B

dans laquelle :

- A est O, S, NH ou N-CN, NH pouvant être salifié par un acide organique ou inorganique physiologiquement acceptable ;
- n est 1 ou 2 ;
- B est un groupe COOH ou un sel de sodium, potassium, ammonium, calcium ou magnésium ;

et caractérisés en ce que R est un groupe hétérocyclique, monocyclique ou bicyclique,

. le groupe monocyclique étant choisi dans le groupe constitué par les radicaux :

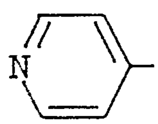

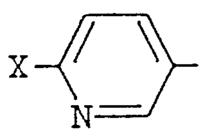

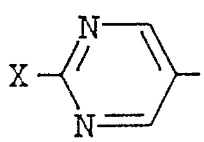

dans lesquels X est :

Cl,

CN et

$NO_2$,

. le groupe bicyclique étant choisi dans le groupe constitué par les radicaux :

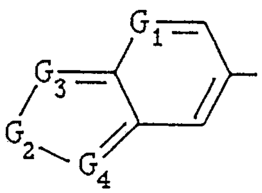

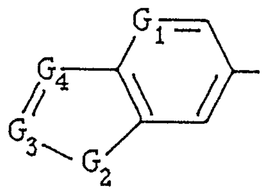

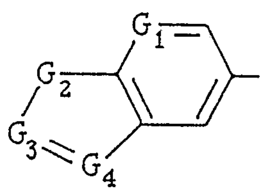

dans lesquels :
$G_1$ est CH ou N,
$G_2$ est $CH_2$, CO, NH, $NC_3$, O ou S,
$G_3$ et $G_4$ sont CH, $CCH_3$, N ou NO.

2. Agents édulcorants selon la revendication 1 dans lesquels :
- R est un groupe 2-cyanopyrid-5-yle ou 2-cyanopyrimidin-5-yle ;
- A est O ou NH, NH pouvant être salifié par HCl ;
- n est 1 quand A est NH et n est 2 quand A est O.

3. Procédé pour édulcorer les aliments, boissons, confiseries, pâtisseries, chewing gum, produits d'hygiène, cosmétiques, articles de toilette, produits pharmaceutiques et vétérinaires, caractérisé en ce qu'il consiste à ajouter à ces produits une quantité adéquate d'au moins un agent édulcorant selon la revendication 1.

4. Préparations édulcorées suivant le procédé de la revendication 3.

5. Compositions édulcorantes caractérisées en ce qu'elles comprennent une quantité adéquate d'un ou plusieurs agents édulcorants selon la revendication 1 et un support ou agent de charge choisi dans le groupe comprenant le polydextrose, l'amidon, les maltodextrines, la cellulose, la méthylcellulose, la carboxyméthylcellulose, l'hydroxyméthylcellulose, la cellulose microcristalline, l'alginate de sodium, les pectines, les gommes, le lactose, le maltose, le glucose, la leucine, le glycérol, le mannitol, le sorbitol, le bicarbonate de sodium et les acides phosphorique, citrique, tartrique, fumarique, benzoïque, sorbique et propionique, et leurs sels de sodium, potassium et calcium, et leurs équivalents.

6. Compositions édulcorantes caractérisées en ce qu'elles comprennent un agent édulcorant selon la revendication 1 et au moins un autre agent édulcorant, l'autre agent édulcorant étant choisi dans le groupe comprenant le saccharose, le sirop de maïs, le fructose, l'aspartame, l'alitame, la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium ou calcium, l'acide cyclamique et ses sels de sodium, potassium, ammonium ou calcium, le trichlorogalactosucrose, la monelline, la thaumatine et leurs équivalents.

**Revendications pour l'Etat contractant suivant:**

1. Procédé pour la préparation d'agents édulcorants de formule :

$$R-NH-\overset{A}{\overset{\|}{C}}-NH-(CH_2)_n-B$$

dans laquelle :
- A est O, S, NH ou N-CN, NH pouvant être salifié par un acide organique ou inorganique physiologiquement acceptable ;
- n est 1 ou 2 ;

- B est un groupe COOH ou un sel de sodium, potassium, ammonium, calcium ou magnésium ;
- R est un groupe hétérocyclique, monocyclique ou bicyclique,
. le groupe monocyclique étant choisi dans le groupe constitué par les radicaux :

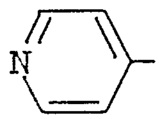

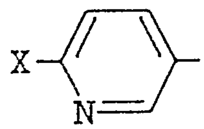

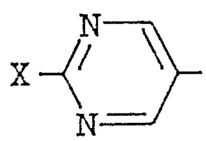

dans lesquels X est :
Cl,
CN et
$NO_2$,
. le groupe bicyclique étant choisi dans le groupe constitué par les radicaux :

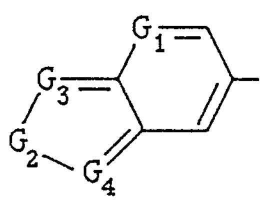

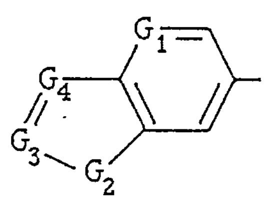

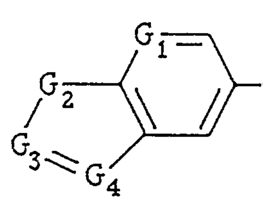

dans lesquels :
$G_1$ est CH ou N,
$G_2$ est $CH_2$, CO, NH, $NCH_3$, O ou S,
$G_3$ et $G_4$ sont CH, $CCH_3$, N ou NO ;
caractérisé en ce qu'il consiste à combiner un composé de formule $H_2N-(CH_2)_n-$ B avec un composé de formule R-N=C=A quand A est O ou S, à condenser un composé de formule R-NH-C(=S)-NH-$(CH_2)_n$-

B avec $H_2NCN$ quand A est NCN, et à hydrolyser un composé de formule R-NH-C(=NCN)-NH-$(CH_2)_n$- B quand A est NH,
R, A, n et B correspondant aux définitions données ci-dessus.

2. Procédé selon la revendication 1, caractérisé en ce que la combinaison avec R-N=C=A est effectuée dans l'eau à température ambiante.

3. Procédé selon la revendication 1, caractérisé en ce que la condensation avec $H_2NCN$ est effectuée en présence de dicyclohexylcarbodiimide.

4. Procédé selon la revendication 1, caractérisé en ce que l'hydrolyse du groupe NCN est réalisée par une solution aqueuse de HCl à 70 ° C.

5. Procédé selon la revendication 1, caractérisé en ce que dans les composés utilisés :
- R est un groupe 2-cyanopyrid-5-yle ou 2-cyanopyrimidin-5-yle ;
- A est O ou NH, NH pouvant être salifié par HCl ;
- n est 1 quand A est NH et n est 2 quand A est O.

Office européen des brevets

# RAPPORT DE RECHERCHE EUROPEENNE

Numero de la demande

EP 88 42 0421

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 195 730 (UNIVERSITE CLAUDE BERNARD)<br>* Revendications 1-8 * | 1-5 | C 07 D 213/75<br>C 07 D 239/28<br>C 07 D 271/12<br>A 23 L 1/236 |
| A | EP-A-0 195 731 (UNIVERSITE CLAUDE BERNARD)<br>* Revendications 1-10 * | 1-5 | |
| A | EP-A-0 107 597 (UNIVERSITE CLAUDE BERNARD)<br>* Revendications 2-8 * | 1-5 | |

DOMAINES TECHNIQUES RECHERCHES (Int. Cl.4)

C 07 D
A 23 L

Le présent rapport a été établi pour toutes les revendications

| Lieu de la recherche | Date d'achèvement de la recherche | Examinateur |
|---|---|---|
| LA HAYE | 11-03-1989 | VAN MOER A.M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)